# EUROPEAN PATENT APPLICATION

(11) **EP 3 636 321 A1**
(43) Date of publication of application: **15.04.2020**
(21) Application number: 18199694.3
(22) Date of filing: 10.10.2018
(51) Int. Cl.: A61Q 5/06, A61K 8/87, C08G 18/08, C08G 18/10, C08G 18/42, C09D 175/06

(54) **POLYURETHANE UREA DISPERSIONS AT LEAST PARTIALLY ORIGINATED FROM RENEWABLE SOURCES AND THEIR PRODUCTION AND USES**

(71) Applicant: Covestro Deutschland AG, 51373 Leverkusen (DE)
(72) Inventor: The designation of the inventor has not yet been filed
(74) Representative: Levpat

(57) **Abstract**

The present invention relates to aqueous polyurethane urea dispersions based on polyester polyols, which dispersions result in low emissions and are used as coating compositions.

## Description

The present invention relates to aqueous polyurethane urea dispersions based on polyester polyols that comprises components which are at least partially originated from renewable sources, their use in cosmetics as well as their production process.

Aqueous polyurethane urea dispersions (PUDs) have become established as film formers in cosmetic compositions. There are a lot of examples of PUDs which comprise components which are generated solely from oil based chemicals, which are also called based on non-renewable sources. However, because of the rising CO₂ value in the air an increasing need of applying sustainable components in industry in general, but in particular in daily life application as cosmetics is needed. So far, polyurethane urea dispersions which are based on polyester polyols on the basis of adipic acid are used to prepare film formers in cosmetics. Examples of these are described in WO 2016/116403 A1, WO 2009/11813 A1 or in WO 2009/118105 A1 in different cosmetic applications. However, none of these are based on components which are originated from renewable sources.

Therefore, there is a need for components of PU film formers based at least partly on renewable sources.

One aim of the invention was to diminish at least a part of the obstacles of the prior art. A further aim was to provide polyurethane urea dispersions with a reduced carbon fraction that stems from non-renewable components. A further aim was to provide polyurethane urea dispersions with an increased carbon fraction that stems from renewable sources. It was additionally an aim of the invention to provide different applications of sustainable cosmetics. Further was it an aim to provide a process to produce sustainable PUs.

According to a first aspect of the invention an aqueous polyurethane urea dispersion is provided containing polyurethane urea polymers composed of polyester polyols based on succinic acid. The polyurethane urea polymers are composed of at least the following components:
a) from 5 to 40 wt.-% of at least one aliphatic or aromatic polyisocyanate having a functionality ≥ 2,
b) from 40 to 90 wt.-% of at least one polyester polyol, having a functionality ≥ 2 and a molar mass Mₙ of from 400 to 8000 g/mol, composed of succinic acid and at least one dihydroxy compounds whose carbon skeleton contains from 2 to 12 carbon atoms,
c) from 0.1 to 5 wt.-% of at least one isocyanate-reactive compound having a functionality ≥ 2 which contains potentially ionic groups,
d) from 0.1 to 17 wt.-% of at least one polyamine having a molar mass Mₙ of from 32 to 400 g/mol and a functionality of from 1 to 3,
e) optionally from 0 to 15 wt.-% of at least one non-ionic, isocyanate-reactive hydrophilising agent,
f) optionally from 0 to 7.0 wt.-% of at least one polyhydroxy compound having a molar mass Mₙ < 400 g/mol and a functionality of from 2 to 4,
g) optionally from 0 to 8.0 wt.-% of at least one neutralising agent for neutralising the potentially ionic groups of component c),
wherein the sum of components a) to g) is less than or equally to 100 wt.-% and
wherein at least 50 wt.-% of the components utilized to compose the polyurethane urea polymers, especially of components a) to g), are originated from renewable sources according to ISO 16128-1:2016, part 1.

The expression "originated from renewable sources" according to the invention means that for the respective material a material source is selected where more than 90 wt.-%, or preferably more than 95 wt.-%, or preferably more than 99 wt.-% of the material that is called "originated from renewable sources" stems from plants or a fermentation process, where only living organisms and plants are involved in the fermentation process to build the renewable source. Basis for the definition of the natural origin of these components can also be found in ISO 16128-1:2016, part 1, page 2.

Preference is given to polyurethane urea polymers composed of at least
a) from 7.5 to 40 wt.% of at least one aliphatic or aromatic polyisocyanate having a functionality ≥ 2,
b) from 45 to 85 wt.% of at least one polyester polyol having a functionality ≥ 2 and a number-average molar mass Mn of from 400 to 8000 g/mol, composed of succinic acid and at least two different dihydroxy compounds whose carbon skeleton contains from 2 to 12 carbon atoms,
c) from 0.4 to 4.5 wt.% of at least one isocyanate-reactive compound having a functionality ≥ 2 which contains potentially ionic groups,
d) from 0.25 to 15 wt.% of at least one polyamine having a molar mass of from 32 to 400 g/mol and a functionality of from 1 to 3,
e) optionally from 0 to 15 wt.% of at least one non-ionic, isocyanate-reactive hydrophilising agent,
f) optionally from 0 to 7.0 wt.% of at least one polyhydroxy compound having a molar mass n < 400 g/mol and a functionality of from 2 to 4,
g) optionally from 0 to 8.0 wt.% of at least one neutralising agent for neutralising the potentially ionic groups of component c),
wherein the sum of components a) to g) is less than or equal to 100 wt.%.

Particular preference is given to polyurethane urea polymers composed of
a) from 10 to 30 wt.% of at least one aliphatic or aromatic polyisocyanate having a functionality ≥ 2,
b) from 50 to 80 wt.% of at least one polyester polyol having a functionality ≥ 2 and a number-average molar mass Mn of from 400 to 8000 g/mol, composed of succinic acid and at least two different dihydroxy compounds whose carbon skeleton contains from 2 to 12 carbon atoms,
c) from 0.75 to 4.0 wt.% of at least one isocyanate-reactive compound having a functionality ≥ 2 which contains potentially ionic groups,
d) from 0.7 to 12 wt.% of at least one polyamine having a molar mass of from 32 to 400 g/mol and a functionality of from 1 to 3,
e) optionally from 0 to 15 wt.% of at least one non-ionic, isocyanate-reactive hydrophilising agent,
f) optionally from 0 to 7.0 wt.% of at least one polyhydroxy compound having a molar mass < 400 g/mol and a functionality of from 2 to 4,
g) optionally from 0 to 8.0 wt.% of at least one neutralising agent for neutralising the potentially ionic groups of component c),
wherein the sum of components a) to g) is less than or equal to 100 wt.%.

The number-average molar mass Mₙ of the polyester polyols is determined by gel permeation chromatography against a polystyrene standard in tetrahydrofuran at 23°C.

The polyester polyol used according to the invention has preferably a glass transition temperature Tg in the range from -80°C to 0°C, particularly preferably in the range from -70°C to -10°C, determined by DSC measurement according to DIN 65467 at a heating rate of 20 K/minute.

Suitable polyisocyanates a) are the aromatic, araliphatic, aliphatic or cycloaliphatic polyisocyanates known *per se* to the person skilled in the art. Suitable polyisocyanates a) are, for example, 1,4-butylene diisocyanate, 1,5-pentamethylene diisocyanate, 1,6-hexamethylene diisocyanate (HDI), isophorone diisocyanate (IPDI), 2,2,4- and/or 2,4,4-trimethylhexamethylene diisocyanate, the isomeric bis-(4,4'-isocyanatocyclohexyl)methanes or mixtures thereof of any desired isomer content, 1,4-cyclohexylene diisocyanate, 1,4-phenylene diisocyanate, 2,4- and/or 2,6-toluene diisocyanate or hydrogenated 2,4- and/or 2,6-toluene diisocyanate, 1,5-naphthalene diisocyanate, 2,4'- or 4,4'-diphenylmethane diisocyanate, 1,3- and 1,4-bis-(2-isocyanato-prop-2-yl)-benzene (TMXDI), 1,3-bis(isocyanatomethyl)benzene (XDI), (S)-alkyl 2,6-diisocyanatohexanoates or (L)-alkyl 2,6-diisocyanatohexanoates.

The proportionate use of polyisocyanates having a functionality > 2 is also possible. These include modified diisocyanates having a uretdione, isocyanurate, urethane, allophanate, biuret, iminooxadiazinedione and/or oxadiazinetrione structure, as well as unmodified polyisocyanates having more than 2 NCO groups per molecule, for example 4-isocyanatomethyl-1,8-octane diisocyanate (nonane triisocyanate) or triphenylmethane-4,4',4"-triisocyanate.

Preference is given to polyisocyanates or polyisocyanate mixtures of the above-mentioned type having solely aliphatically and/or cycloaliphatically bonded isocyanate groups having a mean functionality of from 2 to 4, preferably from 2 to 2.6 and particularly preferably from 2 to 2.4.

Polyester polyols which can be used as compounds b) have a number-average molecular weight Mₙ of from 400 to 8000 g/mol, preferably from 400 to 6000 g/mol and particularly preferably from 400 to 3000 g/mol. The number-average molecular weight Mₙ of the polyester polyols is determined by gel permeation chromatography against a polystyrene standard in tetrahydrofuran at 23°C. Their hydroxyl number according to DIN ISO 4629 is from 22 to 400 mg KOH/g, preferably from 30 to 300 mg KOH/g and particularly preferably from 40 to 250 mg KOH/g, and they have an OH functionality of from 1 to 6, preferably from 1.5 to 4.0, particularly preferably from 1.9 to 2.1.

The polyester polyols b) are prepared according to the prior art preferably by polycondensation from succinic acid, succinic anhydride and/or succinic acid esters with two different dihydroxy compounds having molar masses of from 62 to 300 g/mol, having from 2 to 12 carbon atoms and functionalities of at least 2, which can be branched or unbranched and the hydroxyl groups of which are primary or secondary. The dihydroxy compounds can also contain ether groups. Preferably, the dihydroxy compounds are selected from the group consisting of ethylene glycol, 1,2-propanediol, 1,3-propanediol, 1,4-butanediol, 2,3-butanediol, 2-methyl-1,3-propanediol, 1,5-pentanediol, neopentyl glycol, 1,6-hexanediol, 3-methyl-1,5-pentanediol, 1,8-octanediol, 1,10-decanediol, 1,2-dodecanediol, diethylene glycol, triethylene glycol, dipropylene glycol, tripropylene glycol and oligo-tetrahydrofurans having hydroxyl end groups. Particularly preferably, the two dihydroxy compounds are selected from diols having a carbon skeleton of from three to six carbon atoms, most particularly preferably from the group consisting of hexanediol, neopentyl glycol and 1,3-propanediol.

The preparation of the polyester polyols b) is carried out according to the prior art at elevated temperature in the range from 150 to 250°C and with the application of a vacuum of from 1 to 100 mbar and/or under a stream of nitrogen, optionally using an esterification or transesterification catalyst, the reaction being completed to such an extent that the acid number according to DIN EN ISO 2114 is below a value of 5 mg KOH/g.

Succinic acid for the preparation of component b) can be obtained from regenerative and renewable sources, the preparation process frequently proceeding *via* a fermentation process of starch or biomass, as disclosed, for example, in DE 10 2008 051727 A1 and DE 10 2007 019184. Preferably, at least 50 wt.-% of the succinic acid is based on renewable sources. Furthermore or additionally, at least a part of the at least one dihydroxy compounds of component b) could be originated from renewable sources and therefore increase the carbon fraction that stems from renewable sources of the polyurethane urea dispersion according to the invention. Preferably, at least one dihydroxy compounds component b) is selected from 1,4-butandiol, 1,3-propandiol, isopropandiol, 1,6-hexandiol, ethylene glykol, which are originated from renewable sources or mixtures of at least two thereof.

Component c) contains potentially ionic groups, which can be of either cationic or anionic nature. Compounds having a cationic, anionic or non-ionic dispersing action are those which contain, for example, sulfonium, ammonium, phosphonium, carboxylate, sulfonate, phosphonate groups or groups which can be converted into the above-mentioned groups by salt formation (potentially ionic groups) or polyether groups and can be incorporated into the macromolecules by isocyanate-reactive groups that are present. Preferred suitable isocyanate-reactive groups are hydroxyl and amine groups.

Suitable ionic or potentially ionic compounds c) are, for example, mono- and di-hydroxycarboxylic acids, mono- and di-aminocarboxylic acids, mono- and di-hydroxysulfonic acids, mono- and di-aminosulfonic acids as well as mono- and di-hydroxyphosphonic acids or mono- and di-aminophosphonic acids and their salts, such as dimethylolpropionic acid, dimethylolbutyric acid, hydroxypivalic acid, N-(2-aminoethyl)-β-alanine, 2-(2-amino-ethylamino)-ethanesulfonic acid, ethylenediamine-propyl- or -butyl-sulfonic acid, 1,2- or 1,3-propylenediamine-β-ethylsulfonic acid, malic acid, citric acid, glycolic acid, lactic acid, glycine, alanine, taurine, lysine, 3,5-diaminobenzoic acid, an addition product of IPDI and acrylic acid (EP-A 0 916 647, Example 1) and its alkali and/or ammonium salts; the adduct of sodium bisulfite with 2-butene-1,4-diol, polyether sulfonate, the propoxylated adduct of 2-butenediol and NaHSO₃ described, for example, in DE-A 2 446 440 (pages 5-9, formulae I-III), as well as structural units which can be converted into cationic groups, such as N-methyl-diethanolamine as hydrophilic chain-extension components. Preferred ionic or potentially ionic compounds are those which have carboxy or carboxylate and/or sulfonate groups and/or ammonium groups and have a functionality of from 1.9 to 2.1. Particularly preferred ionic compounds have an amine functionality of from 1.9 to 2.1 and contain sulfonate groups as ionic or potentially ionic groups, such as the salts of N-(2-aminoethyl)-β-alanine, of 2-(2-amino-ethylamino)-ethanesulfonic acid or of the addition product of IPDI and acrylic acid (EP-A 0 916 647, Example 1). Furthermore or additionally, at least a part of component c) could be originated from renewable sources and therefore increase the carbon fraction that stems from renewable sources of the polyurethane urea dispersion according to the invention. Examples of these components c), which are originated from renewable sources are selected from the group consisting of malic acid, citric acid, glycolic acid, lactic acid, glycine, alanine, taurine and lysine or mixtures of at least two thereof.

The polyamines d) used for the chain extension preferably have a functionality of from 1 to 2 and are, for example, di- or poly-amines as well as hydrazides, for example ethylenediamine, 1,2- and 1,3-diaminopropane, 1,4-diaminobutane, 1,6-diaminohexane, isophoronediamine, isomer mixture of 2,2,4- and 2,4,4-trimethylhexamethylenediamine, 2-methylpentamethylenediamine, diethylenetriamine, 1,3- and 1,4-xylylenediamine, α,α,α',α'-tetramethyl-1,3- and -1,4-xylylenediamine and 4,4-diaminodicyclohexylmethane, dimethylethylenediamine, hydrazine or adipic acid dihydrazide.

There come into consideration as component d) in principle also compounds that contain active hydrogen of differing reactivity towards NCO groups, such as compounds which contain, in addition to a primary amino group, also secondary amino groups or, in addition to an amino group (primary or secondary), also OH groups. Examples thereof are primary/secondary amines, such as 3-amino-1-methylaminopropane, 3-amino-1-ethylaminopropane, 3-amino-1-cyclohexylaminopropane, 3-amino-1-methylaminobutane, also alkanolamines such as N-aminoethylethanolamine, ethanolamine, 3-aminopropanol or neopentanolamine.

Preference is given to diethanolamine and/or hydrazine and/or isophoronediamine (IPDA) and/or ethylenediamine. Particular preference is given to hydrazine and/or isophoronediamine and/or ethylenediamine. Most particular preference is given to a mixture of hydrazine and IPDA.

Suitable compounds e) having a non-ionically hydrophilising action are, for example, polyoxyalkylene ethers which contain at least one hydroxy or amino group. Such polyethers contain an amount of from 30 wt.% to 100 wt.% structural units derived from ethylene oxide. There are suitable linear polyethers having a functionality of from 1 to 2, but also compounds of the general formula (I) 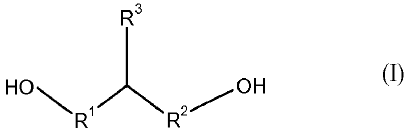 in which
- R¹ and R²: each independently of the other denotes a divalent aliphatic, cycloaliphatic or aromatic radical having from 1 to 18 carbon atoms, which can be interrupted by oxygen and/or nitrogen atoms, and
- R³: represents an alkoxy-terminated polyethylene oxide radical.

Compounds e) having a non-ionically hydrophilising action are, for example, also monohydric polyalkylene oxide polyether alcohols having, in the statistical mean, from 5 to 70, preferably from 7 to 55, ethylene oxide units per molecule, as are obtainable in a manner known *per se* by alkoxylation of suitable starter molecules (e.g. in Ullmanns Encyclopädie der technischen Chemie, 4th Edition, Volume 19, Verlag Chemie, Weinheim p. 31-38).

Suitable starter molecules are, for example, saturated monoalcohols such as methanol, ethanol, n-propanol, isopropanol, n-butanol, isobutanol, sec-butanol, the isomeric pentanols, hexanols, octanols and nonanols, n-decanol, n-dodecanol, n-tetradecanol, n-hexadecanol, n-octadecanol, cyclohexanol, the isomeric methylcyclohexanols or hydroxymethylcyclohexane, 3-ethyl-3-hydroxymethyloxetan or tetrahydrofurfuryl alcohol, diethylene glycol monoalkyl ethers such as, for example, diethylene glycol monobutyl ether, unsaturated alcohols such as allyl alcohol, 1,1-dimethylallyl alcohol or oleic alcohol, aromatic alcohols such as phenol, the isomeric cresols or methoxyphenols, araliphatic alcohols such as benzyl alcohol, anis alcohol or cinnamic alcohol, secondary monoamines such as dimethylamine, diethylamine, dipropylamine, diisopropylamine, dibutylamine, bis-(2-ethylhexyl)-amine, N-methyl- and N-ethyl-cyclohexylamine or dicyclohexylamine as well as heterocyclic secondary amines such as morpholine, pyrrolidine, piperidine or IH-pyrazole. Preferred starter molecules are saturated monoalcohols. Particular preference is given to the use of diethylene glycol monobutyl ether as starter molecule.

Alkylene oxides suitable for the alkoxylation reaction are in particular ethylene oxide and propylene oxide, which can be used in the alkoxylation reaction in any desired sequence or alternatively in admixture.

The number-average molar mass Mₙ of the alkylene oxides is from 300 g/mol to 6000 g/mol, preferably from 500 g/mol to 4000 g/mol and particularly preferably from 750 g/mol to 3000 g/mol, with a functionality of 1. The number-average molecular weight Mₙ of the alkylene oxides is determined by gel permeation chromatography against a polystyrene standard in tetrahydrofuran at 23°C.

Suitable non-ionically hydrophilising, monofunctional compounds e) of that type are, for example, monofunctional alkoxypolyethylene glycols such as, for example, methoxypolyethylene glycols (MPEG Carbowax® 2000 or Methoxy PEG-40, molecular weight range 1800 to 2200, The Dow Chemical Company), monofunctional polyether monoalkyl ethers such as, for example, LB 25 composed of butanol and ethylene oxide as well as propylene oxide, having a mean molar mass Mₙ of 2250 g/mol from Bayer MaterialScience, monofunctional polyether amines (Jeffamine® M 1000, PO/EO molar ratio 3/19 and M 2070, PO/EO molar ratio 10/31, Huntsman Corp.).

There are preferably used as compound e) MPEG Carbowax® 2000, LB 25 or Jeffamine® M 2070. MPEG Carbowax® 2000 or LB 25 are particularly preferred.

The low molecular weight polyols f) which can optionally be used in the formation of the polyurethane resins generally bring about stiffening and/or branching of the polymer chain. The molecular weight is preferably from 62 to 200 and their functionality is preferably from 2 to 3. Suitable polyols c) can contain aliphatic, alicyclic or aromatic groups. Mention may be made here, for example, of the low molecular weight polyols having up to about 20 carbon atoms per molecule, such as, for example, ethylene glycol, diethylene glycol, triethylene glycol, 1,2-propanediol, 1,3-propanediol, 1,4-butanediol, 1,3-butylene glycol, cyclohexanediol, 1,4-cyclohexanedimethanol, 1,6-hexanediol, hydroquinone dihydroxyethyl ether, bisphenol A (2,2-bis(4-hydroxyphenyl)propane), hydrogenated bisphenol A (2,2-bis(4-hydroxycyclohexyl)propane) as well as mixtures thereof, as well as trimethylolpropane, glycerol or pentaerythritol. It is also possible to use ester diols such as, for example, δ-hydroxybutyl-δ-hydroxy-caproic acid ester, ω-hydroxyhexyl-γ-hydroxybutyric acid ester, adipic acid (β-hydroxyethyl) ester or terephthalic acid bis(β-hydroxyethyl) ester. Particular preference is given to hexanediol and/or trimethylolpropane and/or butanediol.

The neutralising agents g) which are optionally to be used for neutralising the potentially ionic groups c) are after or during the preparation of the polyurethane prepolymers from a), b) and c) if it does not contain amino groups as well as optionally from e) and f), if this has not already been carried out in the starting molecules, the partial or complete salt formation of the groups having anionically and/or cationically dispersing action. In the case of anionic groups, there are used for that purpose bases such as ammonia, ammonium carbonate or hydrogen carbonate, trimethylamine, triethylamine, tributylamine, diisopropylethylamine, dimethylethanolamine, diethylethanolamine, triethanolamine, potassium hydroxide or sodium carbonate, preferably triethylamine, triethanolamine, dimethylethanolamine or diisopropylethylamine. The amount of base is from 50 to 100%, preferably from 60 to 90%, of the amount of anionic groups. In the case of cationic groups, sulfuric acid dimethyl ester or succinic acid is used. If only non-ionically hydrophilised compounds with ether groups are used, the neutralisation step is omitted. The neutralisation can also be carried out at the same time as the dispersion if the dispersing water already contains the neutralising agent.

The preparation of the aqueous polyurethane dispersion can be carried out in one or more stages in homogeneous phase or, in the case of a multi-stage reaction, partly in disperse phase. When the polyaddition has been carried out completely or partially, a dispersing, emulsifying or dissolving step takes place. A further polyaddition or modification in disperse phase is then optionally carried out.

All the processes known from the prior art can be used for the preparation of the polyurethane urea dispersions according to the invention, such as the emulsifier/shear force, acetone, prepolymer mixing, melt emulsification, ketimine and solids spontaneous dispersion processes or derivatives thereof. A summary of these methods is to be found in Methoden der organischen Chemie (Houben-Weyl, Erweiterungs- und Folgebände zur 4. Auflage, Volume E20, H. Bartl and J. Falbe, Stuttgart, New York, Thieme 1987, p. 1671-1682). The melt emulsification and the acetone process are preferred. The acetone process is particularly preferred.

Usually, all or some of constituents a) to g), which do not contain primary or secondary amino groups, and one or more polyisocyanates a) are placed in the reactor in order to prepare a polyurethane prepolymer and, optionally diluted with a solvent that is miscible with water but inert towards isocyanate groups, but preferably without a solvent, heated to elevated temperatures, preferably in the range of from 50 to 120°C.

Suitable solvents are, for example, acetone, butanone, ethyl acetate, tetrahydrofuran, dioxane, acetonitrile, dipropylene glycol dimethyl ether and 1-methyl-2-pyrrolidone, which can be added not only at the beginning of the preparation but also later, optionally in portions. Acetone and butanone are preferred. It is possible to carry out the reaction under normal pressure or elevated pressure, for example above the normal pressure boiling temperature of a solvent that is optionally added, such as, for example, acetone.

The catalysts known for accelerating the isocyanate addition reaction, such as, for example, triethylamine, 1,4-diazabicyclo-[2,2,2]-octane, tin dioctoate or dibutyltin dilaurate, can further be placed in the reaction vessel at the same time or added later. Dibutyltin dilaurate is preferred.

Any constituents (a) and/or b) to f), which do not contain primary or secondary amino groups, which were not added at the beginning of the reaction are then metered in. The ratio of isocyanate groups to isocyanate-reactive groups in the preparation of the polyurethane prepolymer is from 0.90 to 4.00, preferably from 1.20 to 3.00, particularly preferably from 1.40 to 2.50. The reaction of components a) with b), c), e) and f) is carried out, based on the total amount of isocyanate-reactive groups, partially or completely, but preferably completely. The degree of conversion is usually monitored by following the NCO content of the reaction mixture. To that end, both spectroscopic measurements, for example infrared or near-infrared spectra, determinations of the refractive index and chemical analyses, such as titrations, of removed samples can be carried out. Polyurethane prepolymers containing free isocyanate groups are obtained without a solvent or in solution.

Then, in a further process step, if this has not yet been carried out or has been carried out only partially, the resulting prepolymer is dissolved with the aid of the above-mentioned solvents.

Possible NH₂- and/or NH-functional components c) and d) are then reacted with the remaining isocyanate groups. This chain extension/termination can be carried out either in solvents prior to the dispersion, during the dispersion or in water after the dispersion. Preferably, the chain extension is carried out prior to the dispersion in water.

The degree of chain extension, that is to say the equivalent ratio of NCO-reactive groups of the compounds used for the chain extension to free NCO groups of the prepolymer is from 40 to 100%, preferably from 60 to 100%, particularly preferably from 70 to 100%.

The amine components c) and d), optionally in solution in water or in a solvent, can be used in the process according to the invention individually or in mixtures, any sequence of addition being possible in principle.

When water or organic solvents are used concomitantly as diluents, the diluent content is preferably from 70 to 95 wt.%.

The preparation of the polyurethane urea dispersion according to the invention from the prepolymers is carried out following the chain extension. To that end, the dissolved and chain-extended polyurethane polymer, optionally with pronounced shear, such as, for example, vigorous stirring, is introduced into the dispersing water or, conversely, the dispersing water is stirred into the polymer solutions. Preferably, the water is added to the dissolved prepolymer.

The solvent still contained in the dispersions after the dispersing step is usually subsequently removed by distillation. Removal during the dispersion is likewise possible.

The solids content of the polyurethane polyurea dispersion according to the invention is from 20 to 70 wt.%, preferably from 30 to 65 wt.% and particularly preferably from 32 to 62 wt.%.

According to a preferred embodiment of the aqueous polyurethane urea dispersion component b) comprises the reaction product of succinic acid with at least one dihydroxy compound whose carbon skeleton contains from 2 to 12 carbon atoms and the succinic acid is originated from renewable sources.

According to a preferred embodiment of the aqueous polyurethane urea dispersion the dihydroxy compound comprises 1,4-butandiol or 1,3-propandiol or both, which are originated from renewable sources.

According to a preferred embodiment of the aqueous polyurethane urea dispersion the polyurethane urea polymers are composed of from 45 to 85 wt.-%, or preferably from 50 to 80 wt.-% of at least one polyester polyol having a functionality ≥ 2 and a molar mass Mₙ of from 400 to 8000 g/mol, or preferably from 500 to 6000 g/mol, or preferably from 600 to 3000 g/mol composed of succinic acid and at least two different dihydroxy compounds whose carbon skeleton contains from 2 to 12 carbon atoms.

According to a preferred embodiment of the aqueous polyurethane urea dispersion the polyurethane urea polymers are composed of from 7.5 to 35 wt.-%, preferably from 10 to 30 wt.-%, or preferably from 15 to 25 wt.-% of at least one aromatic or aliphatic polyisocyanate, preferably of an aliphatic polyisocyanate. Preferably, the polyisocyanate is one of the before mentioned aliphatic polyisocyanates, especially preferably HDI or IPDI.

According to a preferred embodiment of the aqueous polyurethane urea dispersion the polyurethane urea polymers are composed of from 0.5 to 4.5 wt.-%, or preferably from 0.75 to 4.0 wt.-% of at least one isocyanate-reactive compound having a functionality ≥ 2 which contains potentially ionic groups.

According to a preferred embodiment of the aqueous polyurethane urea dispersion the polyurethane urea polymers are composed of from 0.25 to 15.0 wt.-%, preferably from 0.7 to 12 wt.-% of at least one polyamine having a molar mass Mₙ of from 32 to 400 g/mol and a functionality of from 1 to 3, preferably from 1.5 to 2.5..

According to a preferred embodiment of the aqueous polyurethane urea dispersion the polyester polyol has a glass transition temperature Tg in the range from -80°C to 0°C, determined by DSC measurement according to DIN 65467 at a heating rate of 20 K/minute.

According to a further aspect of the invention the use of the aqueous polyurethane urea dispersion according to invention is provided in the production of a cosmetic composition, which comprises the polyurethane urea dispersion in an amount of 0.1 to 90 wt.-%, preferably of 0.5 to 60 wt.-%, or preferably of 1 to 30 wt.-%, based on the total weight of the polyurethane urea dispersion for increasing the carbon fraction from renewable sources of the cosmetic composition. Further constituents and components of the cosmetic composition have already been mentioned in connection with the cosmetic composition according to the invention and are also applicable for the use of the aqueous polyurethane urea dispersion in the cosmetic composition.

According to a further aspect of the invention the use of an aqueous polyurethane urea dispersion for a cosmetic composition selected from the group consisting of a hair cosmetic, a nail polish, a skin cosmetic, a sun protection cosmetic and a color cosmetic, especially to give a good feel and provide water resistance is provided. Further constituents and components of the cosmetic composition have already been mentioned in connection with the cosmetic composition according to the invention and are also applicable for the use of the aqueous polyurethane urea dispersion in the cosmetic composition.

According to a further aspect of the invention a process for preparing a polyurethane urea dispersion based on renewable sources is provided with at least the steps of:
I. Reacting at least the following components:
   a) from 7.5 to 40 wt.-% of at least one aliphatic or aromatic polyisocyanate having a functionality ≥ 2,
   b) from 45 to 85 wt.-% of at least one polyester polyol having a functionality ≥ 2 and a number-average molar mass Mₙ of from 400 to 8000 g/mol, composed of succinic acid and at least two different dihydroxy compounds whose carbon skeleton contains from 2 to 12 carbon atoms,
   c) from 0.4 to 4.5 wt.-% of at least one isocyanate-reactive compound having a functionality ≥ 2 which contains potentially ionic groups,
   d) from 0.25 to 15 wt.-% of at least one polyamine having a molar mass of from 32 to 400 g/mol and a functionality of from 1 to 3,
   e) optionally from 0 to 15 wt.-% of at least one non-ionic, isocyanate-reactive hydrophilising agent,
   f) optionally from 0 to 7.0 wt.-% of at least one polyhydroxy compound having a molar mass Mₙ < 400 g/mol and a functionality of from 2 to 4,
   g) optionally from 0 to 8.0 wt.-% of at least one neutralising agent for neutralising the potentially ionic groups of component c),
wherein the sum of components a) to g) is 100 wt.-% and
wherein at least 50 wt.-% of the components utilized to compose the polyurethane urea polymers, especially of components a) to g), are originated from renewable sources.

According to a preferred embodiment of the process ≥ 80 wt.-%, or preferably ≥ 90 wt.-%, or ≥ 95 wt.-% of the succinic acid of component b) is originated from renewable sources.

According to a preferred embodiment of the process ≥ 50 wt.-%, preferably ≥ 60 wt.-%, or preferably ≥ 60 wt.-%, of at least one of the two different hydroxyl compounds of component b) is originated from renewable sources.

According to a preferred embodiment of the process the hydroxyl compounds is selected from 1,4-butandiol or 1,3-propandiol, which are originated from renewable sources. Preferably 1,4 butandiol originated from renewable sources is used for the composition of the polyurethane urea polymers.

Preferably, the polyurethane produced according to the process according to the invention is utilized to form a cosmetic composition. The quality and quantity of preferable ingredients of the cosmetic composition has already been described above. Further to the mentioned application forms of the cosmetic composition, the cosmetic composition comprising the film forming polyurethane according to the invention is preferably used as a rinse-off or leave-on composition.

The term "rinse-off' according to the invention means that the composition is used in a context whereby the composition is ultimately rinsed or washed from the hair, skin or nail either after or during the application of the product. A "leave-on" product refers to a composition that is applied to hair, skin or nail and not further subjected to a rinsing step. Non-limiting examples of rinse-off products of the present invention include hair conditioner, hair styling rinses, rinse- off hair styling mousse, gels and shampoos.

A further aspect of the invention is a method for shaping hairstyles, stabilizing hair styles, styling nails or treating skin in which a film forming polyurethane according to the invention or produced according to the invention is applied to hair, nails or skin. According to the invention hair also includes eyebrows and eyelashes. Preferably, the method comprises at least the following steps:
I) optionally wetting the hair;
II) optionally cleaning the hair, nails or skin;
III) optionally forming the hair into a hairstyle;
IV) applying the film forming polyurethane or the cosmetic composition to hair, nails or skin;
V) optionally forming the hair into a hairstyle or drying the nail or skin surface.

In the following only the details of steps I) to V) for hairstyling is described as an example. The styling of nails or the skin can be done according to known methods to apply nail varnish to nails or skin car compositions to skin.

In the optional step I) the hair is wetted preferably by rinsing water over the hair or by dipping the hair into water. In the optional step II) the hair is preferably cleaned by a conventional shampoo for at least 1 minute, preferably for at least 2 minutes. In the optional step III) the hair might be formed by different means and into different forms. For example the hair might be curled by hair rollers, by a brush or any other curls forming means. Alternatively, curly hair might be smoothened by an iron. In step IV) the film forming polyurethane or the cosmetic composition comprising the film forming polyurethane is applied to the hair. The application of the film forming polyurethane or the cosmetic composition comprising can be performed by any means the person skilled in the art would select for application thereof to hair. Preferably, the film forming polyurethane or the cosmetic composition is applied to the hair homogeneously. Preferably, after application the film forming polyurethane or the cosmetic composition comprising is left on the hair for a period of from 1 second to 60 minutes, or preferably of from 10 second to 50 minutes, or preferably of from 1 to 20 minutes, or preferably of from 2 to 10 minutes in cases where the film forming polyurethane should be removed. The removing could be done by washing the hair with water or shampoo or any other fluid that is able to wash out the cosmetic composition. In those cases where the film forming polyurethane should stay on the hair as long as possible, at least until the next shampooing of the hair, the cosmetic composition is left on the hair. In optional step V) the hair might be formed preferably as described for step III). Preferably, only one forming step, either step III) or step V) are performed. It is beneficial to apply the film forming polyurethane or the cosmetic composition in step IV) before styling the hair in step V), if the polyurethane or the cosmetic composition should fix the styling for a certain period of time. It is beneficial to apply the film forming polyurethane or the cosmetic composition in step III), if the film forming polyurethane or the cosmetic composition should have the function of a styling aid and help to form the hair into the right position.

Step IV) is preferably performed by using conventional methods of applying the cosmetic composition to the skin, nails, eyebrows or eyelashes, in case the film forming polyurethane is part of a cosmetic composition which is suitable for skin care, nail varnish or eye-make-up like mascara.

Furthermore, a method of styling and/or fixing of hair, especially according to any of steps I) to VI) of the method according to the invention, preferably provokes or supports at least one of the properties when applied to hair:
(i) improving or retaining curl definition of hair;
(ii) imparting humidity resistance to hair;
(iii) reducing hair frizz;
(iv) controlling hair volume;
(v) styling hair;
(vi) straightening hair; or
(vi) improving the appearance of hair, like gloss, touch, color or any other appearance;

The hair treated according to the method according to the invention is preferably natural curly.

The cosmetic composition comprising the film forming polyurethane according to the invention, preferably comprise in particular water and optionally a cosmetically suitable diluent or solvent. The preferred solvents are aliphatic alcohols having C2-4 carbon atoms, such as ethanol, isopropanol, t-butanol, n-butanol; polyol, such as propylene glycol, glycerol, ethylene glycol and polyol ethers; acetone; unbranched or branched hydrocarbons, such as pentane, hexane, isopentane and cyclic hydrocarbons, such as cyclopentane and cyclohexane; and mixtures thereof. A very particularly preferred solvent is ethanol.

Preferably the diluent/solvent, is selected from the group consisting of water, one or more alcohols and mixtures of water and one or alcohols such as the aforementioned aliphatic alcohols.

The cosmetic composition according to the invention preferably comprise solvents/diluents in an amount of 5 to 99.85 wt.-%, advantageously 10 to 95 wt.-%, particularly advantageously 30 to 90 wt.-%, based on the total weight of the composition according to the invention.

Besides the polyurethanes used in accordance with the present invention, the composition according to the invention preferably comprises further suitable film formers. Further film formers are preferably used in cosmetic compositions for applications on hair, especially in hairstyling compositions.

The concentration of one or more further film formers can be from 0 to 20 wt.-% and in particular 0.1 to 10 wt.-%, in each case based on the total weight of the composition.

The film former or film formers are advantageously selected from the group of water-soluble or water-dispersible polyurethanes different from the polyurethanes used according to the invention, the polyureas, silicone resins and/or polyesters, and also the nonionic, anionic, amphoteric and/or cationic polymers and their mixtures.

Advantageous nonionic polymers which may be present in compositions according to the invention alone or in a mixture, preferably also with anionic and/or amphoteric and/or zwitterionic polymers, are selected from:
- polyalkyloxazolines,
- vinyl acetate homopolymers or copolymers. These include, for example, copolymers of vinyl acetate and acrylic acid esters, copolymers of vinyl acetate and ethylene, copolymers of vinyl acetate and maleic acid esters,
- acrylic acid ester copolymers, such as, for example, the copolymers of alkyl acrylate and alkyl methacrylate, copolymers of alkyl acrylate and urethanes,
- copolymers of acrylonitrile and nonionic monomer selected from butadiene and (meth)acrylate,
- styrene homopolymers and copolymers. These include, for example, homopolystyrene, copolymers of styrene and alkyl (meth)acrylate, copolymers of styrene, alkyl methacrylate and alkyl acrylate, copolymers of styrene and butadiene, copolymers of styrene, butadiene and vinylpyridine,
- polyamides,
- vinyllactam homopolymers or copolymers, such as vinylpyrrolidone homo- or copolymers; these include, for example, polyvinylpyrrolidone, polyvinylcaprolactam, copolymers of N-vinylpyrrolidone and vinyl acetate and/or vinyl propionate in various concentration ratios, polyvinylcaprolactam, polyvinylamides and salts thereof, and also copolymers of vinylpyrrolidone and dimethylaminoethyl methacrylate, terpolymers of vinylcaprolactam, vinylpyrrolidone and dimethylaminoethyl methacrylate,
- polysiloxanes,
- homopolymers of N-vinylformamide e.g. PVF from AkzoNobel

Polyurethanes, such as Baycusan ® C2000 available from Covestro Deutschland AG. Particularly preferred nonionic polymers are acrylic acid ester copolymers, homopolymers of vinylpyrrolidone and copolymers, polyvinylcaprolactam.

Very particularly preferred nonionic polymers are homopolymers of vinylpyrrolidone, e.g. Luviskol® K from BASF, copolymers of vinylpyrrolidone and vinyl acetate, e.g. Luviskol® VA grades from BASF or PVPVA® S630L from Ashland, terpolymers of vinylpyrrolidone, vinyl acetate and propionate, such as, for example, Luviskol® VAP from BASF and polyvinylcaprolactams, e.g. Luviskol® PLUS from BASF.

Advantageous anionic polymers are homopolymers or copolymers with monomer units containing acid groups which are optionally copolymerized with comonomers which contain no acid groups. Suitable monomers are unsaturated, free-radically polymerizable compounds which have at least one acid group, in particular carboxylic acid, sulfonic acid or phosphonic acid.

Advantageous anionic polymers comprising carboxylic acid group are:
- Acrylic acid or methacrylic acid homopolymer or copolymer or the salts thereof. These include, for example, the copolymers of acrylic acid and acrylamides and/or sodium salts thereof, copolymers of acrylic acid and/or methacrylic acid and an unsaturated monomer selected from ethylenes, styrene, vinyl esters, acrylic acid esters, methacrylic acid esters, optionally ethoxylated compounds, copolymers of vinylpyrrolidones, acrylic acid and C1-C20 alkyl methacrylates, e.g. Acrylidone® LM from Ashland, copolymers of methacrylic acid, ethyl acrylates and tert-butyl acrylates, e.g. Luvimer® 100 P from BASF.
- Crotonic acid derivative homopolymer or copolymer or the salts thereof. These include, for example, vinyl acetate/crotonic acid, vinyl acetate/acrylate and/or vinyl acetate/vinyl neodecanoate/crotonic acid copolymers e.g. Resyn® 28-1310 or Resyn® 28-2930 of AkzoNobel or Luviset® CAN of BASF, sodium acrylate/vinyl alcohol copolymers,

- Unsaturated C4-C8 carboxylic acid derivatives or carboxylic acid anhydride copolymer selected from copolymers of maleic acid or maleic anhydride or fumaric acid or fumaric anhydride or itaconic acid or itaconic anhydride and at least one monomer selected from vinyl esters, vinyl ethers, vinyl halogen derivatives, phenyl vinyl derivatives, acrylic acid, acrylic acid esters or copolymers of maleic acid or maleic anhydride or fumaric acid or fumaric anhydride or itaconic acid or itaconic anhydride and at least one monomer selected from allyl esters, methallyl esters and optionally acrylamides, methacrylamides, alpha-olefin, acrylic acid esters, methacrylic acid esters, vinylpyrrolidones. Further preferred polymers are methyl vinyl ether/maleic acid copolymers, which are formed by hydrolysis of vinyl ether/maleic anhydride copolymers. These polymers can also be partially esterified (ethyl, isopropyl or butyl esters) or partially amidated.
- Water-soluble or -dispersible anionic polyurethanes, e.g. Luviset® PUR from BASF and Dynamx® from AkzoNobel, Baycusan® C1000, Baycusan ® C1001, Baycusan® C1003, Baycusan® C1004, Baycusan® C1008 from Covestro Deutschland AG, which are different from the polyurethanes according to the invention,
where this list is of course not intended to be limiting.

Advantageous anionic polymers containing sulfonic acid group are salts of polyvinylsulfonic acid, salts of polystyrene sulfonic acid, such as, for example, sodium polystyrene sulfonate or salts of polyacrylamide sulfonic acid.

Particularly advantageous anionic polymers are acrylic acid copolymers, crotonic acid derivative copolymer, copolymers of maleic acid or maleic anhydride or fumaric acid or fumaric anhydride or itaconic acid or itaconic anhydride and at least one monomer selected from vinyl esters, vinyl ethers, vinyl halogen derivatives, phenyl vinyl derivatives, acrylic acid, acrylic acid esters and salts of polystyrene sulfonic acid.

Very particularly advantageous anionic polymers are acrylate copolymers, e.g. Luvimer® from BASF, ethyl acrylate/N-tert-butylacrylamide/acrylic acid copolymers ULTRAHOLD® STRONG from BASF, VA/crotonate/vinyl neodecanoate copolymer, e.g. Resyn™ 28-2930 from AkzoNobel, copolymers such as, for example, copolymers of methyl vinyl ether and maleic anhydride partially esterified e.g. GANTREZ® from Ashland and sodium polystyrene sulfonates e.g. Flexan® 130 from AkzoNobel, as well as RevCare™ NE 100S from Itaconix.

Advantageous amphoteric polymers can be selected from the polymers which contain units A and B distributed randomly in the polymer chain, where A means a unit which is derived from a monomer with at least one basic nitrogen atom, and B is a unit which originates from an acidic monomer which has one or more carboxy groups or sulfonic acid groups, or A and B can be groups which are derived from zwitterionic carboxybetaine monomers or sulfobetaine monomers; A and B can also be a cationic polymer chain which contains primary, secondary, tertiary or quaternary groups, in which at least one amino group carries a carboxy group or sulfonic acid group which is bonded via a hydrocarbon group, or B and C are part of a polymer chain with ethylene-α,β-dicarboxylic acid unit in which the carboxylic acid groups have been reacted with a polyamine which contains one or more primary or secondary amino groups.

Particularly advantageous amphoteric polymers are:
- Polymers which are formed during the copolymerization of a monomer derived from a vinyl compound with carboxy group, such as, in particular, acrylic acid, methacrylic acid, maleic acid, α-chloroacrylic acid, and a basic monomer which is derived from a vinyl compound which is substituted and contains at least one basic atom, such as, in particular, dialkylaminoalkyl methacrylate and acrylate, dialkylaminoalkylmethacrylamide and -acrylamide. Such compounds have been described in the American patent No. 3 836 537.
- Polymers with units which are derived from: a) at least one monomer which is selected from the acrylamides or methacrylamides which are substituted on the nitrogen atom by an alkyl group, b) at least one acidic comonomer which contains one or more reactive carboxy groups, and c) at least one basic comonomer, such as esters of acrylic acid and methacrylic acid with primary, secondary, tertiary and quaternary amino substituents and the quartenization product of dimethylaminoethyl methacrylate with dimethyl sulphate or diethyl sulphate.

N-substituted acrylamides or methacrylamides particularly preferred according to the invention are compounds whose alkyl groups contain 2 to 12 carbon atoms, particularly N-ethylacrylamide, N-t-butylacrylamide, N-t-octylacrylamide, N-octylacrylamide, N-decylacrylamide, N-dodecylacrylamide, and the corresponding methacrylamides.

The acidic comonomers are selected in particular from acrylic acid, methacrylic acid, crotonic acid, itaconic acid, maleic acid, fumaric acid and the alkyl monoesters having 1 to 4 carbon atoms of maleic acid, maleic anhydride, fumaric acid or fumaric anhydride.

Preferred basic comonomers are aminoethyl methacrylate, butylaminoethyl methacrylate, N,N-dimethylaminoethyl methacrylate, N-t-butylaminoethyl methacrylate.
- Crosslinked and completely or partially acylated polyamino amides which are derived from polyamino amides of the following general formula:

   -[CO-R-CO-Z]-
in which R is a divalent group which is derived from a saturated dicarboxylic acid, an aliphatic mono- or dicarboxylic acid with ethylenic double bond, an ester of these acids with a lower alkanol having 1 to 6 carbon atoms or a group which is formed upon the addition of one of these acids onto a bis-primary or bis-secondary amine, and Z is a group which is derived from a bis-primary, mono- or bis-secondary polyalkylenepolyamine, and preferably: a) in quantitative fractions from 60 to 100 mol% the groups -NH-[(CH₂)ₓ-NH-]ₚ- where x = 2 and p = 2 or 3 or x = 3 and p = 2, where this group is derived from diethylenetriamine, triethylenetetramine or dipropylenetriamine; b) in quantitative fractions of from 0 to 40 mol% the group -NH-[(CH₂)ₓ-NH-]ₚ-, in which x = 2 and p = 1, which is derived from ethylenediamine, or the group which originates from piperazine: 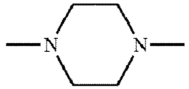 c) in quantitative fractions of from 0 to 20 mol%, the group -H-(CH₂)₆-NH-, which is derived from hexamethylenediamine, where these polyaminoamides are crosslinked by adding a bifunctional crosslinking agent, which is selected from epihalohydrins, diepoxides, dianhydrides and bis-unsaturated derivatives, in an amount of from 0.025 to 0.35 mol of crosslinking agent per amino group of the polyaminoamide, and acylated with acrylic acid, chloroacetic acid or an alkanesultone or salts thereof.

The saturated carboxylic acids are preferably selected from the acids having 6 to 10 carbon atoms, such as adipic acid, 2,2,4-trimethyladipic acid and 2,4,4,-trimethyladipic acid, terephthalic acid; acids with ethylenic double bond, such as, for example, acrylic acid, methacrylic acid and itaconic acid. The alkanesultones used in the acylation are preferably propanesultone or butanesultone, the salts of the acylating agents are preferably the sodium salts or potassium salts.
- Polymers with zwitterionic units of the following formula: 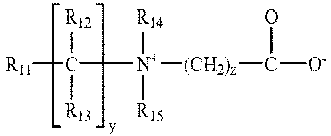 in which R₁₁ is a polymerizable unsaturated group, such as acrylate, methacrylate, acrylamide or methacrylamide, y and z are integers from 1 to 3, R₁₂ and R₁₃ are a hydrogen atom, methyl, ethyl or propyl, R₁₄ and R₁₅ are a hydrogen atom or an alkyl group which is selected such that the sum of the carbon atoms R₁₄ and R₁₅ does not exceed 10.

Polymers which contain such units can also have units which originate from non-zwitterionic monomers, such as dimethyl- and diethylaminoethyl acrylate or dimethyl- and diethylaminoethyl methacrylate or alkyl acrylates or alkyl methacrylates, acrylamides or methacrylamides or vinyl acetate.
- Polymers which are derived from chitosan and contain monomer units which correspond to the following formulae: 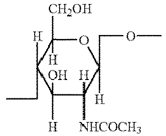 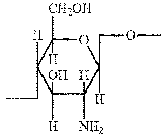 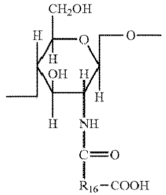 where the first unit is present in quantitative fractions of from 0 to 30%, the second unit is present in quantitative fractions of from 5 to 50% and the third unit is present in quantitative fractions of from 30 to 90%, with the proviso that, in the third unit, R₁₆ is a group of the following formula: 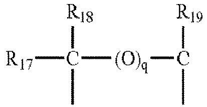 in which: if q = 0, the groups R₁₇, R₁₈ and R₁₉, which are identical or different, are in each case a hydrogen atom, methyl, hydroxy, acetoxy or amino, a monoalkylamine radical or a dialkylamine radical which is optionally interrupted by one or more nitrogen atoms and/or optionally one or more of the groups amino, hydroxy, carboxy, alkylthio, sulfonic acid, alkylthio, whose alkyl group carries an amino radical, where at least one of the groups R₁₇, R₁₈ and R₁₉ is in this case a hydrogen atom; or if q = 1, the groups R₁₇, R₁₈ and R₁₉ are in each case a hydrogen atom, and also the salts which form these compounds with bases or acids.
- Polymers which correspond to the following general formula and which are described, for example, in the French patent 1 400 366: 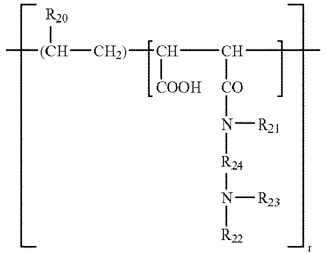 in which R₂₀ is a hydrogen atom, CH₃O, CH₃CH₂O or phenyl, R₂₁ is a hydrogen atom or a lower alkyl group, such as methyl or ethyl, R₂₂ is a hydrogen atom or a lower C₁₋₆-alkyl group, such as methyl or ethyl, R₂₃ is a lower C₁₋₆-alkyl group, such as methyl or ethyl or a group of the formula: -R₂₄-N(R₂₂)₂, where R₂₄ is a group -CH₂-CH₂, -CH₂-CH₂-CH₂- or -CH₂-CH(CH₃)- and where R₂₂ has the meanings given above.
- Polymers which can be formed during the N-carboxyalkylation of chitosan, such as N-carboxymethyl chitosan or N-carboxybutyl chitosan.
- Amphoteric polymers of the type -D-X-D-X, which are selected from:
   a) polymers which are formed through the action of chloroacetic acid or sodium chloroacetate on compounds with at least one unit of the following formula: D-X-D-X,
      in which D is the group 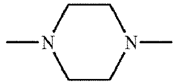 and X is the symbols E or E', where E or E', which are identical or different, are a divalent group, which is a straight-chain or branched alkylene group having up to 7 carbon atoms in the main chain, which is present in unsubstituted form or is substituted by hydroxy groups and can contain one or more oxygen atoms, nitrogen atoms or sulphur atoms and 1 to 3 aromatic and/or heterocyclic rings; where the oxygen atoms, nitrogen atoms and sulphur atoms are present in the form of the following groups: ether, thioether, sulfoxide, sulfone, sulfonium, alkylamine, alkenylamine, hydroxy, benzylamine, amine oxide, quaternary ammonium, amide, imide, alcohol, ester and/or urethane.
   b) Polymers of the formula D-X-D-X, in which D is the group 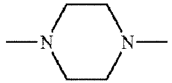 and X is the symbol E or E' and at least once E'; where E has the meanings given above and E' is a divalent group, which is a straight-chain or branched alkylene group having up to 7 carbon atoms in the main chain, which is present in unsubstituted form or is substituted by one or more hydroxy groups and contains one or more nitrogen atoms, where the nitrogen atom is substituted by an alkyl group, which is optionally interrupted by an oxygen atom and obligatorily contains one or more carboxy functions or one or more hydroxy functions and is betainized through reaction with chloroacetic acid or sodium chloroacetate.
- Alkyl(C₁₋₅) vinyl ether/maleic anhydride copolymers which are partially modified by semiamidation with an N,N-dialkylaminoalkylamine, such as N,N-dimethylaminopropylamine or an N,N-dialkylaminoalcohol. These polymers can also contain further comonomers, such as vinylcaprolactam.

Very particularly advantageous amphoteric polymers are, for example, the copolymers octylacrylamide/acrylates/butylaminoethyl methacrylate copolymers which are commercially available under the names AMPHOMER®, AMPHOMER® LV 71 or BALANCE® 47 from AkzoNobel, and methyl methacrylate/methyl dimethylcarboxymethylammonium ethyl methacrylate copolymers.

It is optionally advantageous to neutralize the anionic and amphoteric polymers using suitable bases in order to improve their solubility and/or dispersibility in water.

The following bases can be used as neutralizing agents for polymers which contain acid groups: hydroxides whose cation is an ammonium or an alkali metal, such as, for example, NaOH or KOH.

Other neutralizing agents are primary, secondary or tertiary amines, amino alcohols or ammonia. Preference is given here to 2-amino-2-methyl-1,3-propanediol (AMPD), 2-amino-2-ethyl-1,3-propanediol (AEPD), 2-amino-2-methyl-1-propanol (AMP), 2-amino-1-butanol (AB), 2-amino-1,3-propanediol, monoethanolamine (MEA), diethanolamine (DEA), triethanolamine (TEA), monoisopropanolamine (MIPA), diisopropanolamine (DIPA), triisopropanolamine (TIPA), dimethyllaurylamine (DML), dimethylmyristalamine (DMM) and dimethylstearamine (DMS).

The neutralization can be partial or complete depending on the intended application.

In some cases, it is also possible, but less preferred, to use cationic polymers, such as, for example, polymers which contain primary, secondary, tertiary and/or quaternary amino groups which are bonded as part of the polymer chain or directly to the polymer chain.

Furthermore, the cosmetic composition preferably comprises Biostyle XH of Akzo Nobel, belonging to the group of maltodextrine / VP copolymers.

It is furthermore preferable, if the cosmetic composition comprises shellac

The cosmetic compositions according to the invention can furthermore contain thickeners. Advantageous thickeners are:
- Crosslinked or non-crosslinked acrylic acid or methacrylic acid homo- or copolymers. These include crosslinked homopolymers of methacrylic acid or acrylic acid, copolymers of acrylic acid and/or methacrylic acid and monomers which are derived from other acrylic or vinyl monomers, such as CI0-30 alkyl acrylates, C10-30-alkyl methacrylates and vinyl acetate.
- Thickening polymers of at least partly natural origin, for example based on cellulose, guar gum, xanthan, dehydroxanthan gum, scleroglucan, gellan gum, rhamsan and karaya gum, alginates, maltodextrin, starch and its derivatives, carob seed flour, hyaluronic acid.
- Nonionic, anionic, cationic or amphoteric associative polymers e.g. based on polyethylene glycols and their derivatives, or polyurethanes.
- Crosslinked or non-crosslinked homopolymers or copolymers based on acrylamide or methacrylamide, such as homopolymers of 2-acrylamido-2-methylpropanesulfonic acid, copolymers of acrylamide or methacrylamide and methacryloyloxyethyltrimethylammonium chloride or copolymers of acrylamide and 2-acrylamido-2-methylpropanesulfonic acid are given.

Particularly advantageous thickeners are thickening polymers of natural origin, crosslinked acrylic acid or methacrylic acid homopolymers or copolymers and crosslinked copolymers of 2-acrylamido-2-methylpropanesulphonic acid.

Very particularly advantageous thickeners are xanthan gum, such as the products supplied under the names Keltrol® and Kelza® by CP Kelco, and guar gum, such as the products available under the name Jaguar® HP from Solvay Novecare.

Further very particularly advantageous thickeners are crosslinked homopolymers of methacrylic acid or acrylic acid which are commercially available from Noveon under the names Carbopol® 940, Carbopol® 941, Carbopol® 980, Carbopol® 981, Carbopol® ETD 2001, Carbopol® EDT 2050, Carbopol® 2984, Carbopol® 5984, Carbopol® Clear, Carbopol® Ultrez 30 and Carbopol® Ultrez 10, from 3V under the names Synthalen® K, Synthalen® L and Synthalen® MS.

Further very particularly advantageous thickeners are crosslinked polymers of acrylic acid or methacrylic acid and a C10-30-alkyl acrylate or C10-30-alkyl methacrylate and copolymers of acrylic acid or methacrylic acid and vinylpyrrolidone. Such copolymers are commercially available, for example, from Noveon under the names Carbopol® 1342, Carbopol® 1382, Carbopol® EDT 2020, Carbopol® Ultrez 20, Carbopol® Ultrez 21, Pemulen™ TR1, Pemulen™ EZ-4U or Pemulen™ TR2 and from Ashland under the names Ultrathix P-100 (INCI: Acrylic Acid/VP Crosspolymer).

Very particular advantageous thickeners are crosslinked copolymers of 2-acrylamido-2-methylpropanesulphonic acid. Such copolymers are available, for example, from Clariant under the names Aristoflex® AVC or Aristoflex® AVS (INCI: Ammonium Acryloyldimethyltaurate/VP Copolymer), Aristoflex® Silk (INCI : Sodium Polyacryloyldimethyltaurate), Aristoflex® BLV or Aristoflex® HMB (INCI: Ammonium Acryloyldimethyltaurate/Beheneth-25 Methacrylate Crosspolymer).

These thickeners are preferably present in the composition in a concentration in the range of from about 0 to 2 wt.-%, preferably 0 to 1 wt.-%, based on the total weight of the composition.

Optionally, conventional additives can likewise be present in the composition, for example in order to impart certain modifying properties to it. These may be for example silicones or silicone derivatives, wetting agents, humectants, softeners such as glycerol, glycol and phthalic esters and ethers, fragrances and perfumes, UV absorbers, actives, dyes, pigments, and other colorants, anticorrosive agents, neutralizing agents, antioxidants, anti-sticking agents, combining agents and conditioning agents, antistatic agents, shine agents, preservatives, proteins and derivatives thereof, amino acids, vitamins, emulsifiers, surface-active agents, viscosity modifiers, thickeners and rheology modifiers, gelling agents, opacifiers, stabilizers, surfactants, sequestrants, complexing agents, pearlizing agents, esthetic boosters, fatty acids, fatty alcohols, triglycerides, botanical extracts, clarifying auxiliaries and film formers.

The cosmetic composition according to the invention can advantageously be in the form of a pump spray, aerosol, gel, mousse, foam, lotion, waxes, pomade, cream, oil, milk, oi-in-water emulsion, or an aqueous solution. Preferably, the cosmetic composition is in form of oil-in-water, silicone-in-water, water-in-oil, water-in-silicone, oil-in-water-in-oil, water-in-oil-in-water emulsion.

The composition according to the invention can advantageously be present in a pump spray or aerosol packaging. They can also advantageously be foamed using a propellant gas. Correspondingly, pump sprays, aerosol packagings and foam dispensers which contain the composition according to the invention are likewise part of the invention.

Preferred propellant gases are hydrocarbons such as propane, isobutane and n-butane, and mixtures thereof. Compressed air, carbon dioxide, nitrogen, nitrogen dioxide and dimethyl ether, as well as mixtures of all of these gases can likewise be used.

The person skilled in the art is naturally aware that there are propellant gases that are nontoxic per se which would in principle be suitable for realizing the present invention in the form of aerosol preparations but which nevertheless should be dispensed with on account of an unacceptable impact on the environment or other accompanying phenomena. These are in particular fluorocarbons and chlorofluorocarbons (CFCs) such as e.g. 1,2-difluoroethane (propellant 152 A).

### Examples

In the following section examples of different applications and properties of an inventive polyurethane (PU) is explained and the results are illustrated in the following figures:
Fig. 1 Results of bending experiments of 5 different polymers in form of a diagram
Fig. 2 Results of bending experiments of 2 different polymers with 3 different concentrations in form of a diagram
Fig. 3 Results of curl retention experiments of 5 different polymer compositions in form of a diagram

### Example 1: Bending of inventive polyurethane (PU) compared to synthetic styling polymers

In a first example the bending behavior of Caucasian hair was tested by the application of 5 different polymers, incorporating the inventive PU.

### Preparation of the hair tresses:

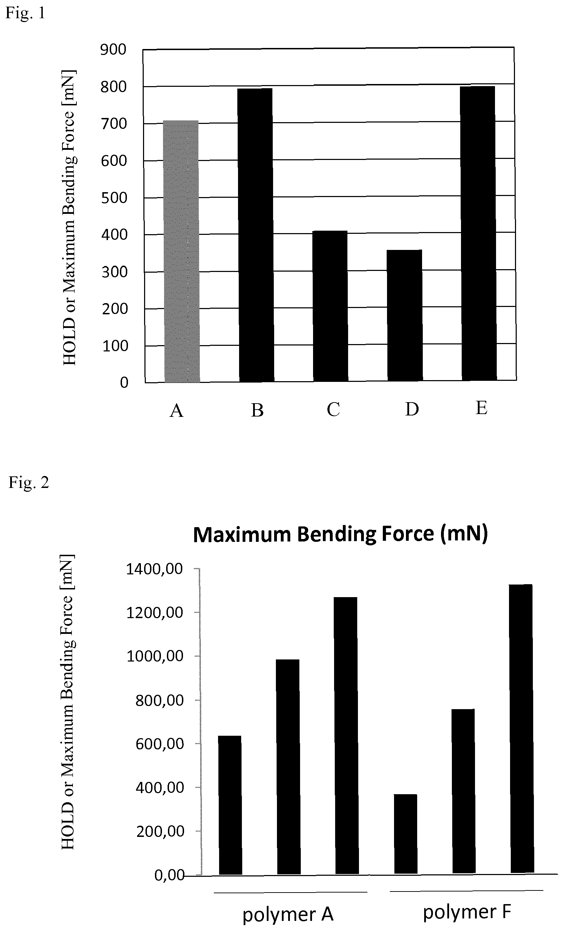

Caucasian virgin hair was shampooed once, carefully rinsed. 1 g of an aqueous polymer solution (2% active) was applied per g of dry hair, corresponding to the integers A, B, C, D and E of Figure 1. The solutions were applied homogeneously on wet hair by combing. The hair was then dried at room temperature and conditioned over night at constant air humidity level (55% Relative humidity).

The hold is measured using a Dia-StronMTT175 apparatus in 3 point bending test configuration. The measurements parameters are set as follows:
Contact Force: 10
Cycles: 1
Maximum Force: 2000
Rate: 10mm/min

The values in Figure 1 are average values of measurement of the maximal bending force (mN) of 5 hair strands. The maximum bending force is the value where the hair breaks in part, meaning that this value is the maximum the hair can hold, why it is called HOLD.

Legend of the x-axis of Figure 1 represents the different polymers as follows:
A: Polyurethane according to the invention (polyurethane-93)
B: polyvinylpyrolidone (INCI:PVP)
C: INCI: VP/methacrylamide/VinylImidazole
D: VP/VA Copolymer
E: Acrylate Copolymer

### Example 2: Bending against a synthetic Polyurethane

In example 2 the aim was to show whether the PUs according to the invention show a different behavior as Pus of the prior art with comparable ingredients.

For this the same hair preparation and test method as in example 1 was performed, except this time the polymer solution contained either 2 wt.-%, 3 wt.-% or 5 wt.-% active of two different polyurethanes, as follow:
A: polyurethane-93, PU-93 (inventive)
F: polyurethane -35, PU-35

Polyurethane-35 is a polyurethane for hair gel having strong hold.

The results of this bending example are shown in Figure 2. It can be seen that the Maximum Bending Force of the inventive PU in the concentration of 2 and 3 wt.-% are higher than for PU-35 and the values of the maximum bending Force of the two PUs in the concentration of 5 wt.-% are nearly the same.

### Example 3: humidity curl retention of natural formula versus formula containing the polyurethane of the invention

Preparation of hair: 0,5 g of the formulation to be tested on 1 g hair, applied on wet hair, hair are rolled around a curled and dried at room temperature overnight under controlled humidity. Obtained hair curls are then placed in a humidity chamber at 90% humidity and 30°C for 3 hours. The length of the curls are measured with a linear.

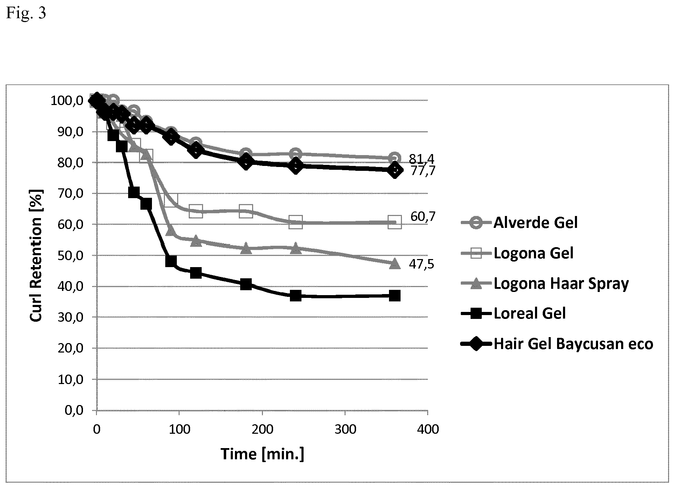

Formulations as shown in Figure 3, in the order of the legend of Figure 3: from Company: Alverde Naturkosmetik™ with the product name Styling Gel (Bio-Lotusblüte), from Company: Logona™ Naturkosmetik with the product name Styling Haargel (Bambus starker Halt (Bio)), from Company: Logona™ Naturkosmetika with the product name STARKER HALT HAARSPRAY (BIO-HOPFEN), from Company L'ORÉAL® with the product name STUDIO LINE SPURENLOS FX Styling Gel 8 (24h Ultra starker Halt). These products are commercially available products. The last formulation in the legend of Figure 2 is Hair Gel Baycusan® eco which comprises the inventive polyurethane in form of PU-93 in an amount of 2 wt.-%. The results in Figure 3 show that the inventive PU has the highest curl retention affect together with the Alverde Gel which does not comprise any components which are originated from renewable sources, especially no polyurethane urea which components are originated from renewable sources at least partially.

## Claims

1. An aqueous polyurethane urea dispersion containing polyurethane urea polymers composed of at least the following components:
a) from 5 to 40 wt.-% of at least one aliphatic or aromatic polyisocyanate having a functionality ≥ 2,
b) from 40 to 90 wt.-% of at least one polyester polyol having a functionality ≥ 2 and a molar mass Mₙ of from 400 to 8000 g/mol, composed of succinic acid and at least one dihydroxy compounds whose carbon skeleton contains from 2 to 12 carbon atoms,
c) from 0.1 to 5 wt.-% of at least one isocyanate-reactive compound having a functionality ≥ 2 which contains potentially ionic groups,
d) from 0.1 to 17 wt.-% of at least one polyamine having a molar mass Mₙ of from 32 to 400 g/mol and a functionality of from 1 to 3,
e) optionally from 0 to 15 wt.-% of at least one non-ionic, isocyanate-reactive hydrophilising agent,
f) optionally from 0 to 7.0 wt.-% of at least one polyhydroxy compound having a molar mass Mₙ < 400 g/mol and a functionality of from 2 to 4,
g) optionally from 0 to 8.0 wt.-% of at least one neutralising agent for neutralising the potentially ionic groups of component c), wherein the sum of components a) to g) is 100 wt.-% and wherein at least 50 wt.-% of the components utilized to compose the polyurethane urea polymers, especially of components a) to g), are originated from renewable sources.

2. The aqueous polyurethane urea dispersion according to claim 1, wherein component b) comprises the reaction product of succinic acid with at least one dihydroxy compound whose carbon skeleton contains from 2 to 12 carbon atoms and the succinic acid is originated from a renewable source.

3. The aqueous polyurethane urea dispersion according to claim 1 or 2, wherein the dihydroxy compound comprises 1,4-butandiol or 1,3-propandiol or both, which are originated from renewable sources.

4. The aqueous polyurethane urea dispersion according to any of the preceding claims, wherein the polyurethane urea polymers are composed of from 45 to 85 wt.-% of at least one polyester polyol having a functionality ≥ 2 and a molar mass Mₙ of from 400 to 8000 g/mol, composed of succinic acid and at least two different dihydroxy compounds whose carbon skeleton contains from 2 to 12 carbon atoms.

5. The aqueous polyurethane urea dispersion according to any of the preceding claims, wherein the polyurethane urea polymers are composed of from 7.5 to 35 wt.-% of at least one aromatic or aliphatic polyisocyanate.

6. The aqueous polyurethane urea dispersion according to any of the preceding claims, wherein the polyurethane urea polymers are composed of from 0.5 to 4.5 wt.-% of at least one isocyanate-reactive compound having a functionality ≥ 2 which contains potentially ionic groups.

7. The aqueous polyurethane urea dispersion according to any of the preceding claims, wherein the polyurethane urea polymers are composed of from 0.25 to 15.0 wt.-% of at least one polyamine having a molar mass Mₙ of from 32 to 400 g/mol and a functionality of from 1 to 3.

8. The aqueous polyurethane urea dispersion according to any of the preceding claims, wherein the polyester polyol has a glass transition temperature Tg in the range from -80°C to 0°C, determined by DSC measurement according to DIN 65467 at a heating rate of 20 K/minute.

9. Use of an aqueous polyurethane urea dispersion according to any of the preceding claims, in the production of a cosmetic composition, which comprises the polyurethane urea dispersion in an amount of 0.1 to 90 wt.-%, preferably of 0.5 to 60 wt.-%, or preferably of 1 to 30 wt.-%, based on the total weight of the polyurethane urea dispersion for increasing the carbon fraction from renewable sources of the cosmetic composition.

10. Use of an aqueous polyurethane urea dispersion according to any of the preceding claims for a cosmetic selected from the group consisting of a hair cosmetic, a nail polish, a skin cosmetic, a sun protection cosmetic and a color cosmetic, especially to give a good feel and provide water resistance.

11. Process of preparing a polyurethane urea dispersion based on renewable sources with at least the steps of:
I. Reacting at least the following components:
a) from 7.5 to 40 wt.-% of at least one aliphatic or aromatic polyisocyanate having a functionality ≥ 2,
b) from 45 to 85 wt.-% of at least one polyester polyol having a functionality ≥ 2 and a number-average molar mass Mₙ of from 400 to 8000 g/mol, composed of succinic acid and at least two different dihydroxy compounds whose carbon skeleton contains from 2 to 12 carbon atoms,
c) from 0.4 to 4.5 wt.-% of at least one isocyanate-reactive compound having a functionality ≥ 2 which contains potentially ionic groups,
d) from 0.25 to 15 wt.-% of at least one polyamine having a molar mass of from 32 to 400 g/mol and a functionality of from 1 to 3,
e) optionally from 0 to 15 wt.-% of at least one non-ionic, isocyanate-reactive hydrophilising agent,
f) optionally from 0 to 7.0 wt.-% of at least one polyhydroxy compound having a molar mass Mₙ < 400 g/mol and a functionality of from 2 to 4,
g) optionally from 0 to 8.0 wt.-% of at least one neutralising agent for neutralising the potentially ionic groups of component c),
wherein the sum of components a) to g) is 100 wt.-% and
wherein at least 50 wt.-% of the components utilized to compose the polyurethane urea polymers, especially of components a) to g), are originated from renewable sources.

12. The process according to claim 11, wherein ≥ 80 wt.-% of the succinic acid of component b) is originated from renewable sources.

13. The process according to claim 11 or 12, wherein ≥ 50 wt.-% of at least one of the two different hydroxyl compounds of component b) is originated from renewable sources.

14. The process according to claim 13, wherein the hydroxyl compounds is selected from 1,4-butandiol or 1,3-propandiol, which are originated from renewable sources.

15. A method for shaping hairstyles, stabilizing hair styles, styling nails or treating skin in which an aqueous polyurethane urea dispersion according to any of claims 1 to 8 or produced according to one of claims 11 to 14 is applied to hair, nails or skin.
